(19) Europäisches Patentamt
European Patent Office
Office européen des brevets

(11) EP 0 880 564 B1

(12) **FASCICULE DE BREVET EUROPEEN**

(45) Date de publication et mention de la délivrance du brevet:
**03.05.2000 Bulletin 2000/18**

(21) Numéro de dépôt: **97905191.9**

(22) Date de dépôt: **12.02.1997**

(51) Int Cl.[7]: **C09D 17/00**, C09C 1/36, C01G 23/053

(86) Numéro de dépôt international:
**PCT/FR97/00266**

(87) Numéro de publication internationale:
**WO 97/30130 (21.08.1997 Gazette 1997/36)**

(54) **PARTICULES DE DIOXYDE DE TITANE**

TITANDIOXIDTEILCHEN

TITANIUM DIOXIDE PARTICLES

(84) Etats contractants désignés:
**AT BE CH DE ES FR GB GR IE IT LI NL SE**

(30) Priorité: **15.02.1996 FR 9601850**

(43) Date de publication de la demande:
**02.12.1998 Bulletin 1998/49**

(73) Titulaire: **RHODIA CHIMIE**
**92408 Courbevoie Cédex (FR)**

(72) Inventeurs:
- **CHOPIN, Thierry**
  **F-95320 Saint-Leu-la-Forêt (FR)**
- **DUPUIS, Dominique**
  **F-95170 Deuil-la-Barre (FR)**
- **WILLEMIN, Claudie**
  **F-75008 Paris (FR)**

(74) Mandataire: **Dubruc, Philippe et al**
**RHODIA SERVICES**
**Direction de la Propriété Industrielle**
**25, quai Paul Doumer**
**92408 Courbevoie Cédex (FR)**

(56) Documents cités:

| | |
|---|---|
| **EP-A- 0 335 773** | **EP-A- 0 351 270** |
| **EP-A- 0 393 857** | **EP-A- 0 401 045** |
| **EP-A- 0 748 624** | **US-A- 4 923 682** |

- **DATABASE WPI Week 8310 Derwent Publications Ltd., London, GB; AN 83-23326K XP002017278 & JP 58 013 668 A (HORUBEIN KOGYO) , 26 Janvier 1983**
- **DATABASE WPI Week 8414 Derwent Publications Ltd., London, GB; AN 84-084520 XP002017279 & JP 59 033 364 A (SANYO SHIKISO) , 23 Février 1984**
- **DATABASE WPI Week 9412 Derwent Publications Ltd., London, GB; AN 94-098009 XP002017280 & JP 06 049 388 A (ISHIHARA SANGYO KAISHA) , 22 Février 1994**
- **DATABASE WPI Week 9403 Derwent Publications Ltd., London, GB; AN 94-022607 XP002033214 & JP 05 330 825 A (ISHIHARA SANGYO KAISHA) , 14 Décembre 1993**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne de nouvelles particules de dioxyde de titane anatase présentant des propriétés anti-UV, utilisables notamment dans les formulations cosmétiques.

Il est connu d'utiliser le dioxyde de titane en tant qu'agent anti-UV dans de nombreuses applications en particulier en cosmétique, peinture, plastique, ...

Dans ces applications, le dioxyde de titane se présente généralement sous forme de particules de taille inférieure à 100 nm en dispersion dans une phase aqueuse ou organique.

Le problème issu de l'utilisation de ces dispersions de particules de dioxyde de titane tient au fait que ces dernières sont souvent instables. Pour contrôler leur stabilité, il est connu de leur ajouter des agents dispersants, en général des polymères organiques.

Cependant, l'ajout de ces agents dispersants n'est pas une solution sans inconvénient. En effet, lorsque la dispersion de dioxyde de titane est mélangée à d'autres produits pour préparer une formulation en cosmétique, peinture ou plastique, cet agent peut présenter des incompatibilités avec l'application (stabilité, agglomération, toxicité, ...) ou les autres composants de la formule.

Dans ces applications, le besoin se fait donc sentir de pouvoir disposer de dispersions de particules de dioxyde de titane stables sans ajout d'agent dispersant.

Un but de la présente invention est donc de proposer des dispersions de particules de dioxyde de titane stables ne contenant pas d'agent dispersant.

Dans ce but, l'invention concerne des particules de dioxyde de titane anatase de taille d'au plus 100 nm, lesdites particules étant recouvertes au moins partiellement d'une couche d'au moins un oxyde, hydroxyde ou oxohydroxyde métallique et présentant une surface spécifique BET d'au moins 70 $m^2/g$ et une densité de l'ordre de 2,2.

Dans ce but, l'invention concerne également le procédé de préparation de ces particules dans lequel on précipite au moins un oxyde, hydroxyde ou oxohydroxyde métallique à la surface de particules de dioxyde de titane anatase de taille d'au plus 100 nm, présentant une surface spécifique BET d'au moins 200 $m^2/g$ et une densité de l'ordre de 2,5.

Enfin, l'invention concerne aussi l'utilisation de ces particules en tant qu'agent anti-UV dans les formulations pour cosmétiques, peintures ou vernis, et dans les plastiques ; et plus particulièrement une composition cosmétique anti-UV comprenant des particules selon l'invention, en quantité telle que la teneur de ladite composition en dioxyde de titane est d'au moins 1 %, de préférence d'au plus 25 % en poids.

Les dispersions de particules selon l'invention présentent en outre l'avantage d'être stables sur une large gamme de pH sans ajout d'agent dispersant.

Elles peuvent également présenter des extraits secs élevés tout en restant stables et en présentant une viscosité faible, notamment inférieure à 1000 mPa.s.

De plus, on observe que ces dispersions conservent le même indice de dispersion même lorsqu'elles ont été mélangées avec les composants d'une formulation par exemple cosmétique.

D'autres caractéristiques, détails et avantages de l'invention apparaîtront plus clairement à la lecture de la description et des exemples qui vont suivre.

L'invention concerne tout d'abord des particules de dioxyde de titane anatase de taille d'au plus 100 nm, lesdites particules étant recouvertes au moins partiellement d'une couche d'au moins un oxyde, hydroxyde ou oxohydroxyde métallique et présentant une surface spécifique BET d'au moins 70 $m^2/g$ et une densité de l'ordre de 2,2.

Les particules selon l'invention sont à base de dioxyde de titane de structure cristalline majoritairement anatase. "Majoritairement" signifie que le taux d'anatase des particules de dioxyde de titane du revêtement est supérieur à 50 % en masse. De préférence, les particules du revêtement présentent un taux d'anatase supérieur à 80 %. Le taux de cristallisation et la nature de la phase cristalline sont mesurés par diffraction RX.

Le diamètre moyen de ces particules est d'au plus 100 nm, de préférence d'au moins 25 nm, encore plus préférentiellement comprise entre 50 et 70 nm. Ce diamètre est mesuré par microscopie électronique par transmission (MET).

Les particules selon l'invention présentent une surface spécifique BET d'au moins 70 $m^2/g$, de préférence d'au moins 100 $m^2/g$.

On entend par surface spécifique BET, la surface spécifique déterminée par adsorption d'azote conformément à la norme ASTMD 3663-78 établie à partir de la méthode BRUNAUER - EMMETT - TELLER décrite dans le périodique "The Journal of the American Society", 60, 309 (1938). Pour mesurer la surface spécifique des particules selon l'invention, lorsqu'elles se présentent sous forme de dispersion, il est essentiel de suivre le protocole de mesure qui consiste à éliminer la phase liquide de la dispersion puis à sécher les particules sous vide à une température de 150°C pendant au moins 4 heures.

Les particules selon l'invention présentent également une densité de l'ordre de 2,2. Par "de l'ordre", on entend que la densité est de 2,2 ± 0,2. Une telle valeur de densité est faible par rapport à la densité classique du dioxyde de

titane anatase qui est de 3,8. Cette densité est mesurée par picnométrie.

Les particules selon l'invention sont recouvertes au moins partiellement d'une couche minérale à base d'au moins un oxyde, hydroxyde ou oxohydroxyde métallique. Ces oxydes, hydroxydes ou oxohydroxydes métalliques peuvent être en particulier choisis parmi $SiO_2$, $ZrO_2$, les oxydes, hydroxydes ou oxohydroxydes de l'aluminium, de zinc, de titane ou d'étain sous forme simple ou mixte. Par mixte, on entend un composé métallique à base d'au moins deux des éléments précités (silicoaluminates, ...).

En général, le rapport en poids du ou des oxydes, hydroxydes ou oxohydroxydes métalliques sur le dioxyde de titane est d'au plus 60 % en poids. Ce rapport est fonction de l'application à laquelle les particules sont destinées. De préférence, lorsque les particules sont utilisées en application cosmétique, ce rapport est d'au plus 25 %, encore plus préférentiellement d'au plus 20 %.

Cette quantité d'oxyde, hydroxyde ou oxohydroxyde métallique est mesurée sur les particules en dispersion par fluorescence X.

Selon le mode préféré de l'invention, les particules sont recouvertes au moins partiellement d'une couche de silice et/ou d'un oxyde, hydroxyde ou oxohydroxyde d'aluminium sous forme simple ou mixte.

Selon une variante préférée, les particules sont recouvertes d'une couche de silice et d'hydroxyde ou oxohydroxyde d'aluminium dans des teneurs en poids de 30 % de $SiO_2$ et 15 % d'$Al_2O_3$ par rapport au dioxyde de titane.

Selon une variante encore plus préférée, les particules sont recouvertes d'une couche de silice et d'hydroxyde ou oxohydroxyde d'aluminium dans des teneurs en poids de 15 % de $SiO_2$ et 5 % d'$Al_2O_3$ par rapport au dioxyde de titane sont particulièrement intéressantes.

Selon le mode préféré de l'invention, les particules se présentent sous la forme d'une dispersion.

En général, cette dispersion présente une conductivité d'au plus 3 msiemens.

Cette dispersion peut présenter une proportion de solide en suspension (extrait sec) comprise entre 10 et 60 % en poids, de préférence d'au moins 35 %, encore plus préférentiellement d'au moins 40 %.

Les dispersion de particules selon l'invention présentant un extrait sec d'au moins 35 % ont l'avantage d'être peu visqueuses, ainsi leur viscosité est en général d'au plus 1000 mPa.s.

Cette dispersion présente, en général, un indice de dispersion des particules dans la phase liquide d'au plus 0,5.

L'indice de dispersion est déterminé par la formule :

$$I = \frac{\varnothing_{84} - \varnothing_{16}}{2\varnothing_{50}}$$

dans laquelle :

- $\varnothing_{84}$ est le diamètre des particules pour lequel 84% des particules ont un diamètre inférieur à $\varnothing_{84}$,
- $\varnothing_{16}$ est le diamètre des particules pour lequel 16% des particules ont un diamètre inférieur à $\varnothing_{16}$,
- $\varnothing_{50}$ est le diamètre moyen des particules.

Les diamètres utiles à la détermination de l'indice de dispersion sont mesurés par sédimentation centrifuge des particules de la dispersion, suivie par rayons X, à l'aide d'un appareil Brookhaven type XDC.

Un tel indice traduit la bonne dispersibilité des particules. Dans le cas des dispersions aqueuses, cet indice est obtenu sur une large gamme de pH pouvant varier de 5,5 à 10. Les dispersions sont stables, elles conservent cette valeur d'indice dans le temps, malgré l'absence d'agent dispersant.

Les particules selon l'invention peuvent également être agglomérées et se présenter sous forme d'une poudre. La taille des agglomérats peut être comprise entre 1 et 40 µm, mesurée par MET.

Suite à un traitement organique, cette poudre peut présenter une bonne redispersibilité dans l'eau ou en milieu organique. Ce traitement organique peut être réalisé, par exemple, par atomisation en présence d'un acide gras tel que l'acide stéarique, d'un sel métallique d'un acide gras, ou encore par greffage d'un trialcoxysilane, ...

L'invention concerne également le procédé de préparation de ces particules qui consiste à précipiter au moins un oxyde, hydroxyde ou oxohydroxyde métallique à la surface de particules de dioxyde de titane anatase de taille d'au plus 100 nm, présentant une surface spécifique BET d'au moins 200 $m^2/g$ et une densité de l'ordre de 2,5.

Cette précipitation peut être réalisée par :

. introduction, dans une dispersion de particules de dioxyde de titane présentant les caractéristiques ci-dessus définies, de précurseurs des oxydes, hydroxydes ou oxohydroxydes métalliques en général sous forme de solutions aqueuses de sels, puis,
. modification du pH pour obtenir la précipitation de ces oxydes, hydroxydes ou oxohydroxydes sur les particules

de dioxyde de titane.

En général, on effectue cette précipitation à une température d'au moins 50°C.

Dans le cas de la précipitation de silice et d'un hydroxyde ou oxohydroxyde d'aluminium, la précipitation peut être réalisée à pH acide ou basique. Le pH est contrôlé par l'ajout d'un acide tel que l'acide sulfurique ou par l'introduction simultanée et/ou alternative d'un composé alcalin du silicium et d'un composé acide de l'aluminium. De préférence, dans ce cas, le pH est compris entre 8 et 10.

On peut précipiter la silice à partir d'un sel de silicium tel qu'un silicate alcalin.

L'hydroxyde ou oxohydroxyde d'aluminium peut être précipité à partir d'un sel d'aluminium tel que le sulfate d'alumine, l'aluminate de soude, le chlorure basique d'aluminium, l'hydroxyde d'aluminium diacétate.

On peut, après la précipitation, récupérer et laver les particules obtenues à la suite du traitement avant de les remettre en dispersion. Cette étape peut être réalisée par centrifugation et lavage ou, de préférence, par lavage par ultrafiltration. Le pH de l'eau de lavage est avantageusement de l'odre de 5,5. Puis, les particules sont redispersées dans un autre milieu liquide de manière à obtenir une dispersion de particules de dioxyde de titane. Ce milieu liquide peut être acide ou basique, de préférence, il s'agit une solution basique présentant un pH de l'ordre de 8-9.

Pour obtenir une poudre de particules selon l'invention, on sèche la dispersion issue du procédé, en général à une température inférieure à 110°C.

Les particules de dioxyde de titane anatase de départ doivent présenter une taille d'au plus 100 nm, une surface spécifique BET d'au moins 200 $m^2$/g et une densité de l'ordre de 2,5.

Les particules de départ sont à base de dioxyde de titane de structure cristalline majoritairement anatase, ainsi que défini précédemment.

Le diamètre moyen de ces particules est d'au plus 100 nm, de préférence d'au moins 25 nm, encore plus préférentiellement compris entre 50 et 70 nm. Ce diamètre est mesuré par microscopie électronique par transmission (MET).

Les particules de départ présentent une surface spécifique BET d'au moins 200 $m^2$/g, de préférence d'au moins 250 $m^2$/g.

Cette surface spécifique BET est mesurée de la même manière que définie précédemment.

Les particules de départ présentent également une densité de l'ordre de 2,5. Par "de l'ordre", on entend que la densité est de 2,5 ± 0,2. Cette densité est donnée par la formule suivante :

$$\text{densité} = \frac{1}{(1/\varrho) + Vi}$$

dans laquelle :

- $\varrho$ est la densité de l'anatase, soit 3,8,
- Vi est le volume apporté par les pores intra particules, il est mesuré par la méthode BJH. On entend par volume mesuré par la méthode BJH, la volume mesuré à partir de la méthode BARRETT-JOYNER-HELENDA décrite dans l'article de l'ouvrage Techniques de l'Ingénieur, et intitulé "Texture des solides poreux ou divisés", p.3645-1 à 3645-13.

Pour mesurer le volume apporté par les pores intra particules des particules selon l'invention, lorsqu'elles se présentent sous forme de dispersion, il est essentiel de suivre le protocole de mesure qui consiste à éliminer la phase liquide de la dispersion puis à sécher les particules sous vide à une température de 150°C pendant au moins 4 heures.

De telles particules peuvent être obtenues par hydrolyse d'au moins un composé du titane A en présence d'au moins un composé B choisi parmi :

(i) les acides qui présentent :

- soit un groupement carboxyle et au moins deux groupements hydroxyles et/ou amines,
- soit au moins deux groupements carboxyles et au moins un groupement hydroxyle et/ou amine,

(ii) les acides phosphoriques organiques de formules suivantes :

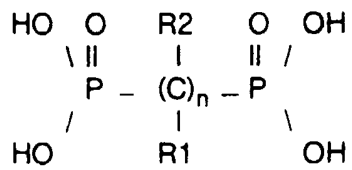

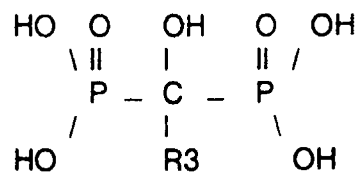

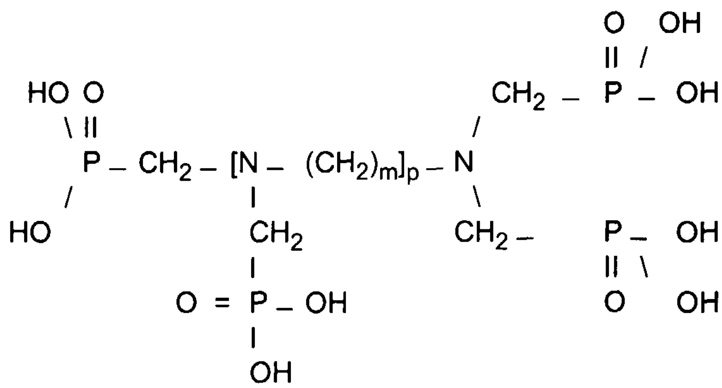

dans lesquelles, n et m sont des nombres entiers compris entre 1 et 6, p est un nombre entier compris entre 0 et 5, R1, R2, R3 identiques ou différents représentant un groupement hydroxyle, amino, aralkyl, aryl, alkyl ou l'hydrogène,
(iii) les composés capables de libérer des ions sulfates en milieu acide,
(iv) les sels des acides décrits ci-dessus,

et en présence de germes de dioxyde de titane anatase présentant une taille d'au plus 8 nm et dans un rapport pondéral exprimé en $TiO_2$ présent dans les germes/titane présent avant introduction des germes dans le milieu d'hydrolyse, exprimé en $TiO_2$, compris entre 0,01 % et 3 %.

La solution de départ, destinée à être hydrolysée, est de préférence totalement aqueuse ; éventuellement on peut ajouter un autre solvant, un alcool par exemple, à condition que le composé du titane A et le composé B utilisés soient alors substantiellement solubles dans ce mélange.

En ce qui concerne le composé du titane A, on utilise en général un composé choisi parmi les halogénures, les oxyhalogénures, les alcoxydes de titane, les sulfates et plus particulièrement les sulfates synthétiques.

On entend par sulfates synthétiques des solutions de sulfates de titanyle réalisées par échange d'ions à partir de solutions de chlorure de titane très pures ou par réaction d'acide sulfurique sur un alcoxyde de titane.

De préférence, on opère avec des composés du titane du type halogénure ou oxyhalogénure de titane. Les halogénures ou les oxyhalogénures de titane plus particulièrement utilisés dans la présente invention sont les fluorures, les chlorures, les bromures et les iodures (respectivement les oxyfluorures, les oxychlorures, les oxybromures et les oxyiodures) de titane.

Selon un mode particulièrement préféré, le composé du titane est l'oxychlorure de titane $TiOCl_2$.

La quantité de composé de titane A présente dans la solution à hydrolyser n'est pas critique.

La solution initiale contient en outre au moins un composé B tel que défini précédemment. A titre d'exemples non limitatifs de composés B entrant dans le cadre de la présente invention, on peut citer notamment :

- les acides hydroxypolycarboxyliques, et plus particulièrement les acides hydroxydi- ou hydroxytricarboxyliques tels que l'acide citrique, l'acide maléique et l'acide tartrique.

- les acides (polyhydroxy)monocarboxyliques, comme par exemple l'acide glucoheptonique et l'acide gluconique,
- les acides poly(hydroxycarboxyliques), comme par exemple l'acide tartrique,
- les monoacides dicarboxyliques et leurs amides correspondantes, comme par exemple l'acide aspartique, l'asparagine et l'acide glutamique,
- les aminoacides monocarboxyliques, hydroxylés ou non, comme par exemple la lysine, la sérine et la thréonine,
- l'aminotriphosphonate de méthylène, l'éthylènediaminotétraphosphonate de méthylène, le triéthylènetétraaminohexaphosphonate de méthylène, le tétraéthylènepentaaminoheptaphosphonate de méthylène, le pentaéthylènehexaaminooctaphosphonate de méthylène,
- le diphosphonate de méthylène; de 1,1' éthylène; de 1,2 éthylène; de 1,1' propylène; de 1,3 propylène; de 1,6 hexaméthylène; le 2,4 dihydroxypentaméthylène - 2,4 diphosphonate; le 2,5 dihydroxyhexaméthylène - 2,5 disphosphonate ; le 2,3 dihydroxybutylène - 2,3 diphosphonate ; le 1 hydroxybenzyle - 1,1' diphosphonate ; le 1 aminoéthylène 1-1' diphosphonate; l'hydroxyméthylène diphosphonate ; le 1 hydroxyéthylène 1,1' diphosphonate ; le 1 hydroxypropylène 1-1' diphosphonate; le 1 hydroxybutylène 1-1' diphosphonate; le 1 hydroxyhexaméthylène - 1,1' diphosphonate.

Comme déjà indiqué, il est également possible d'utiliser à titre de composé B tous les sels des acides précités. En particulier, ces sels sont soit des sels alcalins, et plus particulièrement des sels de sodium, soit des sels d'ammonium.

Ces composés peuvent être choisis aussi parmi l'acide sulfurique et les sulfates d'ammonium, de potassium,...

De préférence, les composés B tels que définis ci-dessus sont des composés hydrocarbonés de type aliphatique. Dans ce cas, la longueur de la chaîne principale hydrocarbonée n'excède pas de préférence 15 atomes de carbone, et plus préférentiellement 10 atomes de carbone. Le composé B préféré est l'acide citrique.

La quantité de composé B n'est pas critique. D'une manière générale, la concentration molaire du composé B par rapport à celle du composé du titane A est comprise entre 0,2 et 10 % et de préférence entre 1 et 5 %.

Enfin la solution de départ comprend des germes de dioxyde de titane utilisés d'une manière spécifique.

Ainsi, les germes de dioxyde de titane utilisés dans la présente invention doivent présenter tout d'abord une taille inférieure à 8 nm, mesurée par diffraction X. De préférence, on utilise des germes de dioxyde de titane présentant une taille comprise entre 3 et 5 nm.

Ensuite, le rapport pondéral du dioxyde de titane présent dans les germes sur le titane présent dans le milieu d'hydrolyse avant introduction des germes - c'est-à-dire apporté par le composé du titane A - et exprimé en $TiO_2$ est compris entre 0,01 et 3 %. Ce rapport peut être préférentiellement compris entre 0,05 et 1,5 %. La réunion de ces deux conditions sur les germes (taille et rapport pondéral) associée au procédé tel que décrit précédemment permet de contrôler précisément la taille finale des particules de dioxyde de titane en associant à un taux de germes une taille de particule. On peut ainsi obtenir des particules dont la taille varie entre 25 et 100 nm.

On utilise des germes de dioxyde de titane sous forme anatase de manière à induire la précipitation du dioxyde de titane sous forme anatase. Généralement, du fait de leur petite taille, ces germes se présentent plutôt sous la forme d'anatase mal cristallisé. Les germes se présentent habituellement sous la forme d'une suspension aqueuse constituée de dioxyde de titane. Ils peuvent être généralement obtenus de manière connue par un procédé de neutralisation d'un sel de titane par une base.

L'étape suivante consiste à réaliser l'hydrolyse de cette solution de départ par tout moyen connu de l'homme du métier et en général par chauffage. Dans ce dernier cas, l'hydrolyse peut de préférence être effectuée à une température supérieure ou égale à 70°C. On peut aussi travailler dans un premier temps à une température inférieure à la température d'ébullition du milieu puis maintenir le milieu d'hydrolyse en palier à la température d'ébullition.

Une fois l'hydrolyse réalisée, les particules de dioxyde de titane obtenues sont récupérées par séparation du solide précipité des eaux mères avant d'être redispersées dans un milieu liquide de manière à obtenir une dispersion de dioxyde de titane. Ce milieu liquide peut être acide ou basique. Il s'agit de préférence d'une solution basique, par exemple d'une solution aqueuse de soude. C'est à partir de cette dispersion que l'étape de précipitation des oxydes, hydroxydes ou oxohydroxydes métalliques sera réalisée.

Selon une variante particulière, après la récupération des particules obtenues à la suite de l'hydrolyse et avant leur remise en dispersion, on neutralise les particules et on leur fait subir au moins un lavage. Les particules peuvent être récupérées par exemple par centrifugation de la solution issue de l'hydrolyse, elles sont ensuite neutralisées par une base, par exemple une solution d'ammoniaque ou de soude, puis on les lave en les redispersant dans une solution aqueuse, enfin les particules sont séparées de la phase aqueuse de lavage. Après éventuellement un ou plusieurs autres lavages du même type, les particules sont remises en dispersion dans une solution acide ou basique.

Ces particules présentent habituellement un degré de pureté élevé, compatible avec une application en cosmétique.

Enfin, l'invention concerne l'utilisation des particules précédemment décrites en tant qu'agent anti-UV. Elles peuvent notamment être utilisées en tant qu'agent anti-UV dans les formulations pour cosmétiques, vernis, peintures

et dans les plastiques.

Introduites dans les formulations cosmétiques, ces dispersions ou poudres de dioxyde de titane permettent d'obtenir un indice SPF (Sun Protection Factor) d'au moins 20.

D'autre part, les formulations obtenues sont photostables, c'est-à-dire qu'elles ne présentent pas de bleuissement après exposition aux UV selon le test défini dans les exemples.

Elles sont particulièrement stables au stockage et on peut constater que les particules de dioxyde de titane conserve leur indice de dispersion dans la formulation.

L'invention concerne des compositions cosmétiques anti-UV comprenant des particules telles que précédemment décrites, en quantité telle que la teneur en dioxyde de titane dans lesdites compositions est d'au moins 1 %, de préférence d'au plus 25 % en poids, encore plus préférentiellement comprise entre 2 et 10 % en poids.

Il est possible d'introduire dans les compositions cosmétiques des particules présentant des tailles de particules différentes.

Les compositions faisant l'objet de l'invention peuvent être formulées en un grand nombre de types de produits, comme les produits solaires type gels, lotions, huiles, crèmes, et plus généralement les produits de maquillage, les autobronzants, les produits de soin, les capillaires, les écrans totaux pour les lèvres et bien d'autres compositions du même type.

On entend par le terme composition ou formulation cosmétique tous les produits ou préparations cosmétiques comme celles décrites dans l'annexe I ("illustrative list by category of cosmetic products") de la directive européenne n° 76/768/CEE du 27 juillet 1976 dite directive cosmétique.

Les compositions cosmétiques faisant l'objet de l'invention peuvent faire appel à un véhicule, ou un mélange de plusieurs véhicules, qui jouent un rôle de diluant, dispersant ou de support pour les autres constituants de la composition et permettent leur répartition lorsque la composition est étalée sur la peau ou les cheveux.

Les véhicules autres que l'eau peuvent être des émolients liquides ou solides, des solvants, des humectants, des épaississants ou des poudres. On peut par exemple utiliser seuls ou en mélange les types de véhicules suivants :

- des émollients tels que l'alcool stéarylique, le glycéryl monoricinoléate, l'alcool oléylique, l'alcool cétylique, l'isopropyl isostéarate, l'acide stéarique, l'isobutyl palmitate, l'isocétyl stéarate , l'isopropyl laurate, l'hexyl laurate, le décyloléate, l'octadécane-2-ol, l'alcool isocétylique, l'alcool eicosanylique, l'alcool behenylique, le cétylpalmitate, les huiles silicones telles que le diméthylpolysiloxne, le di-n-butyl sebacate, l'isopropyl myristate, l'isopropyl palmitate, l'isopropyl stéarate, le butyl stéarate, le polyéthylène glycol, la lanoline, le beurre de coco, l'huile de graine de coton, l'huile d'olive, l'huile de palme, l'huile de colza, l'huile de soja, l'huile de tournesol, l'huile d'avocat, l'huile d'amande, l'huile de sésame, l'huile de noix de coco, l'huile d'arachide, l'huile de castor, l'huile minérale, le myristate de butyl, l'acide isostéarique, l'acide palmitique, l'isopropyl linoléate, le lauryl lactate, le décyl oléate, le pyristyl myristate ;
- des propulseurs tels que : propane, butane, isobutane, diméthyléther, dioxyde de carbone, dioxyde d'azote ;
- des solvants tels que : éthanol, chlorure de méthylène, isopropanol, acétone, éthylène glycol monoéthyl éther, diéthylène glycol monobutyl éther, diéthylène glycol monoéthyl éther, oxyde de diméthylsulfure, diméthylformamide, tétrahydrofurane ;
- des poudres telles que craie, talc, kaolin, amidon, gommes, silice colloïdale, polyacrylate de sodium, smectites de tétraalkyl et/ou trialkyl aryl ammonium, magnésoaluminosilicate chimiquement modifié, montmorillonite organiquement modifié, silicate d'aluminium hydraté, fumée de silice, polycarboxyvinyl, carboxyméthylcellulose de sodium, éthylène glycol monostéarate.

Les compositions selon l'invention comprennent habituellement de 10 à 99 % en poids d'au moins un véhicule tel que décrit précédemment.

De préférence, les compositions selon l'invention se présentent sous la forme d'émulsions, dans lesquelles un composant huileux est présent avec un émulsifiant de manière à former une émulsion huile dans l'eau ou eau dans l'huile, selon la valeur de la balance hydrophile/lipophile (HLB).

Ainsi, les compositions selon l'invention peuvent comprendre un ou plusieurs composants huileux ou composants ayant les propriétés d'une huile.

Il peut s'agir d'huiles végétales ou minérales, telles que celles proposées dans la liste des émollients cidessus. On peut également utilisés des huiles silicones, volatiles ou non, tels que des polydiméthylsiloxanes.

Ces composants huileux peuvent représenter jusqu'à 90 %, de préférence de 10 à 80 %, du volume de la composition.

Les compositions selon l'invention peuvent comprendre également un ou plusieurs émulsifiants. Selon la nature de ces émulsifiants, les compositions se présenteront sous la forme d'une émulsion huile dans l'eau ou eau dans l'huile.

Pour la préparation d'une émulsion type eau dans l'huile, le ou les émulsifiants choisis doivent présenter une

HLB moyenne comprise entre 1 et 6. Pour la préparation d'une émulsion type huile dans l'eau, le ou les émulsifiants choisis doivent présenter une HLB moyenne comprise supérieure à 6. La quantité de ces émulsifiant dans les compositions selon l'invention peuvent varier entre 1 et 50 % en poids, de préférence entre 2 et 20 %.

Ces compositions cosmétiques peuvent aussi contenir des agents tensioactifs qui servent à disperser, émulsionner, solubiliser, stabiliser divers composés utilisés pour leurs propriétés émollientes ou humectantes. Ces agents tensioactifs sont utilisés dans ces compositions à des concentrations variant de 0,05 à 50 % en poids de la préparation. On retrouve ainsi des tensioactifs anioniques, non-ioniques, cationiques, zwitterioniques ou amphotères ou des mélanges de ces tensioactifs, tels que :

- **tensioactifs anioniques :**

  . les alkylesters sulfonates de formule R-CH($SO_3$M)-COOR', où R représente un radical alkyle en $C_8$-$C_{20}$, de préférence en $C_{10}$-$C_{16}$, R' un radical alkyle en $C_1$-$C_6$, de préférence en $C_1$-$C_3$ et M un cation alcalin (sodium, potassium, lithium), ammonium substitué ou non substitué (méthyl-, diméthyl-, triméthyl-, tetraméthylammonium, diméthylpiperidinium ...) ou dérivé d'une alcanolamine (monoéthanolamine, diéthanolamine, triéthanolamine ...). On peut citer tout particulièrement les méthyl ester sulfonates dont les radical R est en $C_{14}$-$C_{16}$ ;

  . les alkylsulfates de formule $ROSO_3$M, où R représente un radical alkyle ou hydroxyalkyle en $C_{10}$-$C_{24}$, de préférence en $C_{12}$-$C_{20}$ et tout particulièrement en $C_{12}$-$C_{18}$, M représentant un atome d'hydrogène ou un cation de même définition que ci-dessus, ainsi que leurs dérivés éthoxylénés (OE) et/ou propoxylénés (OP), présentant en moyenne de 0,5 à 6 motifs, de préférence de 0,5 à 3 motifs OE et/ou OP ;

  . les alkylamides sulfates de formule RCONHR'$OSO_3$M où R représente un radical alkyle en $C_2$-$C_{22}$, de préférence en $C_6$-$C_{20}$, R' un radical alkyle en $C_2$-$C_3$, M représentant un atome d'hydrogène ou un cation de même définition que ci-dessus, ainsi que leurs dérivés éthoxylénés (OE) et/ou propoxylénés (OP), présentant en moyenne de 0,5 à 60 motifs OE et/ou OP ;

  . les sels d'acides gras saturés ou insaturés en $C_8$-$C_{24}$, de préférence en $C_{14}$-$C_{20}$, les alkylbenzènesulfonates en $C_9$-$C_{20}$, les alkylsulfonates primaires ou secondaires en $C_8$-$C_{22}$, les alkylglycérol sulfonates, les acides polycarboxyliques sulfonés décrits dans GB - A - 1 082 179, les sulfonates de paraffine, les N-acyl N-alkyltaurates, les alkylphosphates, les alkyliséthionates, les alkylsuccinamates les alkylsulfosuccinates, les monoesters ou diesters de sulfosuccinates, les N-acyl sarcosinates, les sulfates d'alkylglycosides, les polyéthoxycarboxylates, le cation étant un métal alcalin (sodium, potassium, lithium), un reste ammonium substitué ou non substitué (méthyl-, diméthyl-, triméthyl-, tetraméthylammonium, diméthylpiperidinium ...) ou dérivé d'une alcanolamine (monoéthanolamine, diéthanolamine, triéthanolamine ...) ;

- **des agents tensio-actifs non-ioniques :**

  . les alkylphénols polyoxyalkylénés (polyéthoxyéthylénés, polyoxypropylénés, polyoxybutylénés) dont le substituant alkyle est en $C_6$-$C_{12}$ et contenant de 5 à 25 motifs oxyalkylènes ; à titre d'exemple, on peut citer les TRITON X-45, X-114, X-100 ou X-102 commercialisés par Rohm & Haas Cy.;

  . les glucosamides, glucamides ;

  . les glycérolamides dérivés de N-alkylamines (US-A-5,223,179 et FR-A-1,585,966)

  . les alcools aliphatiques en C8-C22 polyoxyalkylénés contenant de 1 à 25 motifs oxyalkylènes (oxyéthylène, oxypropylène) ; à titre d'exemple, on peut citer les TERGITOL 15-S-9, TERGITOL 24-L-6 NMW commercialisés par Union Carbide Corp., NEODOL 45-9, NEODOL 23-65, NEODOL 45-7, NEODOL 45-4 commercialisés par Shell Chemical Cy., KYRO EOB commercialisé par The Procter & Gamble Cy.

  . les produits résultant de la condensation de l'oxyde d'éthylène avec un composé hydrophobe résultant de la condensation de l'oxyde de propylène avec le propylène glycol, tels les PLURONIC commercialisés par BASF ;

  . les oxydes d'amines tels que les oxydes d'alkyl C10-C18 diméthylamines, les oxydes d'alkoxy $C_8$-$C_{22}$ éthyl dihydroxy éthylamines ;

  . les alkylpolyglycosides décrits dans US-A-4 565 647 et leurs polyoxyalkylénés;

  . les amides d'acides gras en $C_8$-$C_{20}$

  . les acides gras éthoxylés

  . les amides, amines, amidoamines éthoxylées

- **des agents tensio-actifs amphotères et zwitterioniques :**

  . les alkyltriméthylsulfobétaïnes, les produits de condensation d'acides gras et d'hydrolysats de proteines, les alkylampho-propionates ou -dipropionates, les alkylsultaines, les dérivés amphotères des alkylpolyamines

comme l'AMPHIONIC XLâ commercialisé par RHONE-POULENC, AMPHOLAC 7T/Xâ et AMPHOLAC 7C/Xâ commercialisés par BEROL NOBEL sont utilisés pour diminuer l'irritation provoquée par les autres agents tensioactifs, principalement les agents tensioactifs anioniques.

On peut également utiliser un émulsifiant choisi parmi ceux de la liste suivante :

| Nom chimique de l'émulsifiant | Nom commercial | HLB |
|---|---|---|
| trioléate sorbitan | Arlacel 85 | 1,8 |
| tristéarate sorbitan | Span 65 | 2,1 |
| glycerol monooléate | Aldo MD | 2,7 |
| glycerol monostéarate | Atmul 84S | 2,8 |
| glycerol monolaurate | Aldo MC | 3,3 |
| sesquioleate sorbitan | Arlacel 83 | 3,7 |
| monooleate sorbitan | Arlacel 80 | 4,3 |
| monostéarate sorbitan | Arlacel 60 | 4,7 |
| polyoxyéthylène stéaryl éther | Brij 72 | 4,9 |
| polyoxyéthylène sorbitol dérivé de cire d'abeille | G-1702 | 5 |
| polyglyceryl-3 diisostéarate | Plurol Diisostéarique | 6 |
| PEG 200 dilaurate | Emerest 2622 | 6,3 |
| monopalmitate sorbitan | Arlacel 40 | 6,7 |
| PEG 200 monostéarate | Tegester PEG 200 MS | 8,5 |
| sorbitan monolaurate | Arlacel 200 | 8,6 |
| PEG 400 dioléate | Tegester PEG 400-DO | 8,8 |
| polyoxyéthylène (5) monostéarate | Ethofat 60-16 | 9,0 |
| polyoxyéthylène (4) sorbitan monostéarate | Tween 61 | 9,6 |
| polyoxyéthylène (4) lauryléther | Brij 30 | 9,7 |
| polyoxyéthylène (5) sorbitan monooléate | Tween 81 | 10,0 |
| PEG 300 monooléate | Neutronyx 834 | 10,4 |
| polyoxyéthylène (20) sorbitan tristéarate | Tween 65 | 10,5 |
| polyoxyéthylène (20) sorbitan trioléate | Tween 85 | 11,0 |
| polyoxyéthylène monostéarate | Myrj 45 | 11,1 |
| PEG 400 monooléate | Emerest 2646 | 11,7 |
| PEG 400 monostéarate | Tegester PEG 400 | 11,9 |
| polyoxyéthylène 10 monooléate | Ethofat 0/20 | 12,2 |
| polyoxyéthylène 10 stéaryl éther | Brij 76 | 12,4 |
| polyoxyéthylène 10 cétyl éther | Brij 56 | 12,9 |
| polyoxyéthylène (4) sorbitan monolaurate | Tween 21 | 13,3 |
| PEG 600 monooléate | Emerest 2660 | 13,7 |
| PEG 1000 dilaurate | Kessco | 13,9 |
| polyoxyéthylène sorbitol dérivé de la lanoline | G-1441 | 14,0 |
| polyoxyéthylène (12) lauryl éther | Ethosperse LA-12 | 14,4 |
| PEG 1500 dioléate | Pegosperse 1500 | 14,6 |
| polyoxyéthylène (14) laurate | Arosurf HFL-714 | 14,8 |
| polyoxyéthylène (20) sorbitan monostéarate | Tween | 14,9 |
| polyoxyéthylène (20) sorbitan monooléate | Tween 80 | 15,0 |
| polyoxyéthylène (20) stéaryl éther | Brij 78 | 15,3 |
| polyoxyéthylène (20) sorbitan monopalmitate | Tween 40 | 15,6 |
| polyoxyéthylène (20) cétyl éther | Brij 58 | 15,7 |
| polyoxyéthylène (25) oxypropylène | Monostéarate G-2162 | 16,0 |
| polyoxyéthylène (20) sorbitol monolaurate | Tween 20 | 16,7 |
| polyoxyéthylène (23) lauryl éther | Brij 35 | 16,9 |
| polyoxyéthylène (50) monostéarate | Myrj 53 | 17,9 |

(suite)

| Nom chimique de l'émulsifiant | Nom commercial | HLB |
|---|---|---|
| PEG 4000 monostéarate | Pegoperse 4000 MS | 18,7 |

Les compositions selon l'invention peuvent comprendre de l'eau dans une teneur pouvant aller jusqu'à 80 % en volume, de préférence comprise entre 5 et 80 %.

Les compositions selon l'invention peuvent comprendre en outre un agent tensio-actif siliconé de haut poids moléculaire, qui peut être un émulsifiant utilisé à la place de ceux mentionnés ci-dessus.

Cet agent peut être un diméthylpolysiloxane de haut poids moléculaire avec des chaînes polyoxyéthylène et/ou polyoxypropylène ayant un poids moléculaire compris entre 10 000 et 50 000 et de structure :

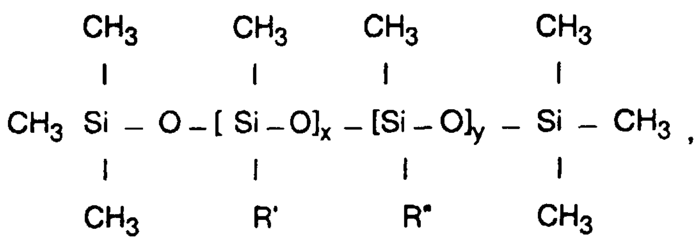

dans laquelle :

- les groupes R' et R" sont choisis parmi H, les alkyl en $C_1$-$C_{18}$ et $[CH_2CH_2O]_a[CH_2CH(CH_3)O]_bH$, l'un des groupes R' et R" peut être le lauryl, l'autre ayant un poids moléculaire sompris entre 1000 et 5000,
- a est compris entre 9 et 115, de préférence entre 10 et 114
- b est compris entre 0 et 50, de préférence entre 0 et 49,
- x est compris entre 133 et 673, de préférence entre 388 et 402
- y est compris entre 25 et 0,25, de préférence entre 15 et 0,75.

Le diméthylpolysiloxane peut être utilisé sous la forme d'une dispersion dans un siloxane volatile, cette dispersion comprenant de 1 à 20 % en volume de diméthylpolysiloxane.

Les diméthylpolysiloxanes peuvent être choisis parmi les cyclométhicone et diméthicone coplyols, tels que DC 3225C de Dow Coming ou le lauryl méthicone copolyol tel que le DC Q2-5200 de Dow Corning.

Il peut s'agir aussi du Mirasil DMCO de Rhône-Poulenc, du cetyl dimethicone copylol Abil AM90 de TH. Goldschmidt AG.

La composition selon l'invention peut comprendre jusqu'à 25 % en poids d'un tel agent tensio-actif.

Les compositions selon l'invention peuvent comprendre également un filtre solaire organique, tel que par exemple :

| Nom CTFA | Nom commercial | Commersialisé par |
|---|---|---|
| Benzophénone-3 | UVINUL M-40 | BASF |
| Benzophénone-4 | UVINUL MS-40 | BASF |
| Benzophénone-8 | SPECRA-SORB UV-24 | American Cyanamid |
| Glyceryl PABA | NIPA GMPA | Nipa Labs |
| Octocrylène | UVINUL N-539 SG | BASF |
| Octyl diméthyl PABA | ESCALOL 507 | ISP |
| Octyl méthoxycinnamate | PARSOL MCX | Givaudan/Roux |
| Octyl salicylate | UVINUL O-18 | BASF |
| PABA | n° 102 | Merck |
| Acide sulfonique 2-phénylbenzimidazole-5 | EUSOLEX 232 | Merck |
| 3-(4-méthylbenzylidène)-Camphre | EUSOLEX 6300 | EM Ind. |
| 4-isopropyl dibenzoyl methane | EUSOLEX 8020 | EM Ind. |
| Butyl méthoxy dibenzoyl methane | PARSOL 1789 | Givaudan/Roux |

(suite)

| Nom CTFA | Nom commercial | Commersialisé par |
|---|---|---|
| Etocrylène | UVINUL N-35 | BASF |

On peut également utiliser en tant que filtre solaire tous les composés autorisés dans la directive Européenne N° 76/768/CEE, et ses annexes.

La composition selon l'invention peut comprendre également des agents solaires inorganiques tels que : de l'oxyde de zinc sous forme de particules de taille moyenne compris entre 1 et 300 nm, de l'oxyde de fer sous forme de particules de taille moyenne compris entre 1 et 300 nm, de la silice sous forme de particules de taille moyenne compris entre 1 et 100 nm.

Les compositions peuvent comprendre aussi des addtitifs tels que :

- des conservateurs par exemple les ester de para-hydroxy-benzoate ;
- des antioxydants tels que le butylhydroxytoluène ;
- des humectants tels que le glycérol, le sorbitol, le dibutylphtalate, la gélatine, les PEG par exemple les PEG 200-600;
- des solutions tampons telles que des mélanges d'acide lactique et de soude ou de triéthanolamine ;
- des cires telles que la cire d'abeille, de paraffine ;
- des extraits de plantes ;
- des agents conservateurs comme les méthyl, éthyl, propyl et butyl esters de l'acide p-hydroxybenzoïque, le benzoate de sodium, le GERMABEN (nom de marque) ou tout agent chimique évitant la prolifération bactérienne ou des moississures et utilisé traditionnellement des les compositions cosmétiques sont généralement introduits dans ces compositions à hauteur de 0,01 à 3 % en poids. La quantité de ces produits est généralement ajustée pour éviter toute prolifération de bactéries, moississures ou levures dans les compositions cosmétiques. Alternativement à ces agents chimiques, on peut parfois utiliser des agents modifiants l'activité de l'eau et augmentant fortement la pression osmotique comme les carbohydrates ou des sels;
- ...

Les compositions cosmétiques faisant l'objet de l'invention peuvent également contenir des résines fixatives. Ces résines fixatives sont généralement présentes à des concentrations comprises entre 0.01 et 10%, préférentiellement entre 0,5 et 5 %. Les résines fixatives constituants des compositions cosmétiques faisant l'objet de l'invention sont préférentiellement choisies parmi les résines suivantes : acrylate/acrylamide copolymère, polyvinyl méthyl éther/maléic anhydride copolymère, vinyl acétate/ acide crotonique copolymère, octylacrylamide/acrylate/butylaminoéthyl méthacrylate copolymère, polyvinylpyrrolidone (PVP), copolymères de polyvinylpyrrolidone et de méthyl méthacrylate, copolymère de polyvinylpyrrolidone et d'acétate de vinyle (VA), alccol polyvinylique, copolymère de l'alcool polyvinylique et d'acide crotonique, copolymère d'alcool polyvinylique et d'anhydride maléique, hydroxypropyl cellulose, hydroypropyl guar, polystyrène sulfonate de sodium, polyvinylpyrrolidone/éthyl méthacrylate/ acide méthacrylique terpolymère, monométhyl éther de poly(méthylvinyl éther - acide maléique), les copolymères polytéréphtale d'éthylène glycol / polyéthylène glycol, les copolymères polytéréphtalate d'éthylène glycol / polyéthylène glycol / polyisophtalate sulfonate de sodium, et leurs mélanges. Les résines fixatives peuvent aussi contenir des motifs polyorganosiloxanes fonctionnalisés greffés comme décrit dans le brevet WO 95/06079.

De manière préférentielle, les résines fixatives seront du type polyvinylpyrrolidone (PVP), copolymères de polyvinylpyrrolidone et de méthyl méthacrylate, copolymère de polyvinylpyrrolidone et d'acétate de vinyle (VA), copolymères polytéréphtale d'éthylène glycol / polyéthylène glycol, copolymères polytéréphtalate d'éthylène glycol / polyéthylène glycol / polyisophtalate sulfonate de sodium, et leurs mélanges.

Ces résines fixatives sont préférentiellement dispersées ou solubilisées dans le véhicule choisi.

Les compositions cosmétiques faisant l'objet de l'invention peuvent également contenir des dérivés polymériques exerçant une fonction protectrice.

Ces dérivés polymériques peuvent être présents en quantités de l'ordre de 0,01 à 10 %, de préférence environ 0,1 à 5 %, et tout particulièrement de l'ordre de 0,2 à 3 % en poids, agents tels que

. les dérivés cellulosiques tels que les hydroxyéthers de cellulose, la méthylcellulose, l'éthylcellulose, l'hydroxypropyl méthylcellulose, l'hydroxybutyl méthylcellulose
. les polyvinylesters greffés sur des troncs polyalkylenes tels que les polyvinylacétates greffés sur des troncs polyoxyéthylènes (EP-A-219 048)
. les alcools polyvinyliques

- les copolymères polyesters à base de motifs ethylène téréphtalate et/ou propylène téréphtalate et polyoxyéthylène téréphtalate, avec un rapport molaire (nombre de motifs) ethylène téréphtalate et/ou propylène téréphalate / (nombre de motifs) polyoxyéthylène téréphtalate de l'ordre de 1/10 à 10/1, de préférence de l'ordre de 1/1 à 9/1, les polyoxyéthylène téréphtalates présentant des unités polyoxyéthylène ayant un poids moléculaire de l'ordre de 300 à 5000, de préférence de l'ordre de 600 à 5000 (US-A-3 959 230, US-A-3 893 929, US-A-4 116 896, US-A-4 702 857, US-A-4 770 666);
- les oligomères polyesters sulfonés obtenus par sulfonation d'un oligomère dérivé de de l'alcool allylique éthoxylé, du diméthyltéréphtalate et du 1,2 propylène diol, présentant de 1 à 4 groupes sulfonés (US-A-4 968 451)
- les copolymères polyesters à base de motifs propylène téréphtalate et polyoxyéthylène téréphtalate et terminés par des motifs éthyles, méthyles (US-A-4 711 730) ou des oligomères polyesters terminés par des groupes alkylpolyéthoxy (US-A-4 702 857) ou des groupes anioniques sulfopolyéthoxy (US-A-4 721 580), sulfoaroyles (US-A-4 877 896)
- les polyesters-polyuréthanes obtenus par réaction d'un polyesters de masse moléculaire en nombre de 300-4000 obtenus à partir d'acide adipique et/ou d'acide téréptalique et/ou d'acide sulfoisophtalique et d'un diol de masse inférieure à 300, sur un prépolymère à groupements isocyanates terminaux obtenus à partir d'un polyoxyéthylène glycol de masse moléculaire de 600-4000 et d'un diisocyanate ((FR-A-2 334 698)
- les monoamines ou polyamines éthoxylées, les polymères d'amines éthoxylées (US-A-4 597 898, EP-A-11 984)
- les oligomères polyesters sulfonés obtenus par condensation de l'acide isophtalique, du sulfosuccinate de diméthyle et de diéthylène glycol (FR-A-2 236 926)

Les performances des compositions cosmétiques faisant l'objet de l'invention peuvent aussi être améliorées par l'emploi d'agents plastifiants. L'agent plastifiant pourra constituer entre 0,1 à 20 % de la formulation de préférence de 1 à 15 %. Parmi les agents plastifiants particulièrement utiles, on peut citer les adipates, les phtalates, les isophtalates, les azélates, les stéarates, les copolyols silicones, les glycols, l'huile de ricin, ou leurs mélanges.
On peut aussi avantageusement ajouter à ces compositions des agents séquestrants des métaux, plus particulièrement ceux séquestrants du calcium comme les ions citrates ou des agents émollients comme les silicones ou des huiles ou corps gras utilisés à ce propos dans l'industrie cosmétique (huiles minérales, esters d'acides gras, triglycérides, silicones, ....).
On peut aussi rajouter des polymères hydrosolubles ou hydrodispersables comme le collagène ou certains dérivés non allergisants de protéines animales ou végétales (hydrolisats de protéines de blé par exemple), des hydrocolloïdes naturels (gomme de guar, de caroube, de tara, .....) ou issus de procédés de fermentation comme la gomme xanthane et les dérivés de ces polycarbohydrates comme les celluloses modifiées (par exemple Hydroxyéthylcellulose, carboxyméthylcellulose), les dérivés du guar ou de la caroube comme leurs dérivés cationiques ou des dérivés nonioniques (par exemple hydroxypropylguar), les dérivés anioniques (carboxyméthylguar et carboxyméthylhydroxypropylguar).
A ces composés, on peut ajouter en association des poudres ou des particules minérales comme du carbonate de calcium, des oxydes minéraux sous forme de poudre ou sous forme colloidale (particules de taille inférieure ou de l'ordre de un micromètre, parfois de quelque dizaines de nanomètres) comme de la silice, des sels d'aluminium utilisés généralement comme antitranspirants, du kaolin, du talc, des argiles et leurs dérivés, ...
A ces ingrédients on rajoute généralement pour augmenter l'agrément lors de l'utilisation de la composition par le consommateur, un ou des parfums, des agents colorants parmi lesquels on peut citer les produits décrits dans l'annexe IV ("list of colouring agents allowed for use in cosmetic products") de la directive européenne n° 76/768/CEE du 27 juillet 1976 dite directive cosmétique et/ou des agents opacifiants comme des pigments.
Finalement, la composition peut aussi contenir des polymères viscosants ou gélifiants, comme les polyacrylates réticulés -CARBOPOL commercialisés par GOODRICH-, les dérivés de la cellulose comme l'hydroxypropylcellulose, la carboxyméthylcellulose, les guars et leurs dérivés, la caroube, la gomme de tara ou de cassia, la gomme xanthane, les alginates, les carraghénannes, les dérivés de la chitine comme le chitosan utilisés seuls ou en association, ou les mêmes composés, généralement sous la fome de polymères hydrosolubles mdifiés par des groupements hydrophobes liés de manière covalente au squelette polymère comme décrit dans le brevet WO 92/16187 et/ou de l'eau pour amener le total des constituants de la formulation à 100 %.
Les compositions cosmétiques faisant l'objet de l'invention peuvent également contenir des agents dispersants polymériques en quantité de l'ordre de 0,1 à 7% en poids, pour contrôler la dureté en calcium et magnésium, agents tels que :

- les sels hydrosolubles d'acides polycarboxyliques de masse moléculaire de l'ordre de 2000 à 100 000, obtenus par polymérisation ou copolymérisation d'acides carboxyliques éthyléniquement insaturés tels que acide acrylique, acide ou anhydride maleique, acide fumarique, acide itaconique, acide aconitique, acide mesaconique, acide citraconique, acide méthylènemalonique, et tout particulièrement les polyacrylates de masse moléculaire de l'ordre

de 2 000 à 10 000 (US-A-3 308 067), les copolymères d'acide arylique et d'anhydride maleique de masse moléculaire de l'ordre de 5 000 à 75 000 (EP-A-66 915)

. les polyéthylèneglycols de masse moléculaire de l'ordre de 1000 à 50 000.

Les exemples suivants illustrent l'invention sans toutefois en limiter la portée.

**EXEMPLES**

**Exemple 1 - Préparation d'une dispersion de particules selon l'invention avec un traitement à base de silice**

Préparation des particules de départ

On ajoute successivement à 1300 g d'une solution d'oxychlorure de titane à 1,73 mol/kg :

- 121 g d'acide chlorhydrique à 36 %,
- 15,14 g d'acide citrique,
- 1562 g d'eau épurée,
- 10,30 g (3,6 % /$TiO_2$) de germes d'anatase présentant une taille comprise entre 5 et 6 nm.

Le mélange est porté à ébullition est y est maintenu pendant 3 h.
La solution est ensuite filtrée et les particules obtenues sont lavées à l'eau jusqu'à élimination complète des chlorures. Elles sont ensuite redispersées à pH 9 (contrôlé par l'ajout de soude) avec un extrait sec de 20 % en poids.
La taille des particules mesurée par MET est de 60 nm. L'analyse par diffraction X indique que les particules sont à base de dioxyde de titane uniquement sous forme anatase.
Leur densité est de 2,52 (Vi = 0,14 cc/g).
La surface spécifique, mesurée par la méthode BET sur les particules de la dispersion séchées à une température de dégazage de 150 °C, est de 300 $m^2$/g.

Traitement des particules par de la silice

On introduit sous agitation 750 g de la dispersion ci-dessus dans 750 g d'eau déionisée.
Le mélange obtenu est transféré dans un réacteur et la température est élevée jusqu'à 90°C. Le pH est ajusté à 9 par l'ajout de soude.
On y introduit de façon continue et simultanée une solution de silicate de sodium (solution à 335 g/l de $SiO_2$) contenant l'équivalent de 30 g de $SiO_2$ et une solution d'acide sulfurique à 80 g/l. Le débit de la solution de silicate alcalin est fixé à 2 ml/min, le pH est régulé à 9 par l'ajout de l'acide sulfurique
Après introduction des réactifs, la température est maintenue à 90 °C pendant 2 h.
Après refroidissement, la dispersion est centrifugée. Le gâteau obtenu est lavé trois fois à l'eau, puis redispersé à pH 8,5 avec un extrait sec de 40 % en poids.

Propriétés des particules obtenues

La taille des particules mesurée par MET est de 60 nm.
La surface spécifique, mesurée par la méthode BET sur les particules de la dispersion séchées sous vide à une température de 150 °C pendant 4 heures, est de 140 $m^2$/g.
Le taux de $SiO_2$, mesuré par fluorescence X, est de 19 % en poids par rapport au dioxyde de titane.
La densité est de 2,2.
La viscosité est de 750 mPa.s.
L'indice de dispersion est de 0,45. Cette dispersion est particulièrement stable : au bout d'un mois, la mesure de l'indice de dispersion reste de 0,45.

**Exemple 2 - Préparation d'une dispersion de particules selon l'invention avec un traitement à base de silice et d'hydroxyde d'aluminium**

Les particules de départ sont les mêmes que dans l'exemple 1.

Traitement des particules par de la silice et de l'oxyde, hydroxyde ou oxohydroxyde d'aluminium

On introduit 750 g de la dispersion de départ dans un réacteur muni d'une agitation. Puis, on ajoute 750 g d'eau épurée et on élève la température jusqu'à 90°C. Le pH de la dispersion est ajusté à 9 par ajout de soude.

On introduit tout d'abord de façon continue et simultanément une solution de silicate de sodium (solution à 335 g/l de $SiO_2$) contenant l'équivalent de 22,5 g de $SiO_2$ et une solution d'acide sulfurique à 80 g/l dans une quantité telle que le pH est maintenu à 9. Le débit de la solution de silicate de sodium est fixé à 2 ml/min. On observe ensuite un temps de mûrissement de 1 h à 90°C.

On introduit ensuite, à pH 9 et à 90°C, de façon continue une solution aqueuse d'aluminate de sodium (solution à 240 g/l en $Al_2O_3$) contenant l'équivalent de 7,5 g de $Al_2O_3$. Le débit de la solution d'aluminate est de 2 ml/min, le pH est régulé à 9 par introduction simultanée d'une solution aqueuse d'acide sulfurique 6N.

Lorsque les réactifs ont été introduits, on effectue un temps de mûrissement de 2 h à 90°C, puis la dispersion est refroidie.

La dispersion obtenue est centrifugée. Le gâteau obtenu est lavé trois fois avec de l'eau puis est redispersé.

Le pH de la dispersion est ajusté à 7,5 par ajout de $H_2SO_4$, elle présente un extrait sec de 30 % en poids.

Propriétés des particules obtenues

La taille des particules mesurée par MET est de 60 nm.

La surface spécifique, mesurée par la méthode BET sur les particules de la dispersion séchées sous vide à une température de 150 °C sous vide, est de 135 $m^2/g$.

Le taux de $SiO_2$, mesuré par fluorescence X, est de 14,9 % en poids, celui de $Al_2O_3$ de 5 %.

La viscosité est de 750 mPa.s.

La densité est de 2,15.

L'indice de dispersion est de 0,45. Cette dispersion est particulièrement stable : au bout d'un mois, la mesure de l'indice de dispersion reste de 0,45.

**Exemple 3 - Stabilité en fonction du pH de la dispersion de l'exemple 2**

On fait varier le pH de la dispersion de l'exemple 2 par ajout d'acide sulfurique ou de soude. Les indices de dispersion associés à chaque pH sont rassemblés dans le tableau suivant.

| pH | indice de dispersion |
|---|---|
| 5,5 | 0,43 |
| 6,5 | 0,35 |
| 7,5 (ex.2) | 0,45 |
| 8 | 0,31 |
| 10 | 0,31 |

On observe que pour un pH variant de 5,5 à 10, l'indice de dispersion reste inférieur à 0,45.

**Exemple 4 - Préparation cosmétique anti-UV**

On prépare une composition cosmétique anti-UV selon l'invention à partir de la dispersion de dioxyde de titane de l'exemple 3 en suivant la formulation suivante :

| CTFA | Ingrédients | % en poids |
|---|---|---|
| cyclométhicone et diphényldiméthicone | Mirasil C-DPDM | 4 |
| caprylic capric triglycéride | Miglyol 812 N | 4 |
| palmitate d'octyl | Crodamol OP | 4 |
| huile minérale | Marcol 82 | 5 |
| copolymère PVP/eicosène | Antaron V 220 | 3 |
| acétate de vitamine E | | 0,3 |
| stéarate de glycéryl | | |

(suite)

| CTFA | Ingrédients | % en poids |
|---|---|---|
| stéarate de propylème glycol<br>isostéarate de glycéryl<br>isostéarate de propylème glycol<br>olleth 25<br>ceteth 25 | Hydrolactol 70 | 10 |
| cecyl phosphate de potassium | Amphisol K | 2 |
| polysorbate 20 | Tween 20 | 1 |
| conservateur | Germaben II | 0,2 |
| allantoine | | 0,2 |
| gomme xanthane | Rhodicare D | 0,2 |
| dispersion exemple 2 | | 15,6 (5% en TiO2) |
| acide lactique | | qs pH = 6,5 |
| eau déionisée | | qs 100 |

Mesure de photostabilité de la composition

La composition cosmétique à tester est introduite via une cellule en quartz dans un appareil Sun-Test de Heraeus et soumise à une énergie E de 500 W/m$^2$ à une température T de 30 °C durant 1 h.
Le contrôle de la photostabilité se fait par observation visuelle de la coloration de la formulation. Visuellement, on n'observe aucun bleuissement.

Mesure de l'indice SPF (Sun Protection Factor) in vitro

Cet indice a été mesuré à l'aide d'un appareil SPF290 d'optométrie selon la méthode décrite dans "Cosmetics & Toiletries", Vol.107, N°10, p.119.
L'indice SPF est 20 ± 2 pour une application de 2 mg/cm$^2$.

Mesure de l'indice de dispersion des particules de titane dans la formulation

L'indice de dispersion est 0,45, c'est-à-dire identique à celui de la dispersion de particules de dioxyde de titane avant formulation.

**Revendications**

1. Particules de dioxyde de titane anatase de taille d'au plus 100 nm, caractérisées en ce que ces particules sont recouvertes au moins partiellement d'une couche d'au moins un oxyde, hydroxyde ou oxohydroxyde métallique et en ce que ces particules présentent une surface spécifique BET d'au moins 70 m$^2$/g et une densité de l'ordre de 2,2.

2. Particules selon la revendication 1, caractérisées en ce que le rapport en poids du ou des oxydes, hydroxydes ou oxohydroxydes métalliques sur le dioxyde de titane est d'au plus 60 % en poids.

3. Particules selon la revendication 1 ou 2, caractérisées en ce qu'elles sont recouvertes au moins partiellement d'une couche de silice ou d'un hydroxyde ou oxohydroxyde d'aluminium sous forme simple ou mixte.

4. Particules selon la revendication 2, caractérisées en ce qu'elles sont recouvertes d'une couche de silice et d'hydroxyde ou oxohydroxyde d'aluminium dans des teneurs en poids de 15 % de $SiO_2$ et 5 % d'$Al_2O_3$ par rapport au dioxyde de titane.

5. Particules selon l'une quelconque des revendications précédentes, caractérisées en ce qu'elles se présentent sous forme de dispersion.

**6.** Particules selon la revendication 5, caractérisées en ce que leur indice de dispersion des particules dans la phase liquide est d'au plus 0,5.

**7.** Particules selon la revendication 5 ou 6, caractérisées en ce que la phase liquide de la dispersion est aqueuse.

**8.** Particules selon la revendication 7, caractérisées en ce que la dispersion présente une conductivité d'au plus 3 msiemens.

**9.** Particules selon l'une quelconque des revendications 5 à 7, caractérisées en ce que l'extrait sec de la dispersion est d'au moins 35 % en poids et en ce que la viscosité de ladite dispersion est d'au plus 1000 mPa.s.

**10.** Particules selon l'une des revendications 1 à 4, caractérisées en ce qu'elles sont agglomérées et se présentent sous forme d'une poudre.

**11.** Procédé de préparation de particules selon l'une des revendications 1 à 10, caractérisé en ce qu'on précipite au moins un oxyde, hydroxyde ou oxohydroxyde métallique à la surface de particules de dioxyde de titane anatase de taille d'au plus 100 nm, présentant une surface spécifique BET d'au moins 200 $m^2/g$ et une densité de l'ordre de 2,5.

**12.** Procédé de préparation selon la revendication 11, caractérisé en ce qu'on précipite au moins un oxyde, hydroxyde ou oxohydroxyde métallique à la surface de particules de dioxyde de titane obtenues par hydrolyse d'au moins un composé du titane A en présence d'au moins un composé B choisi parmi :

(i) les acides qui présentent :

- soit un groupement carboxyle et au moins deux groupements hydroxyles et/ou amines,
- soit au moins deux groupements carboxyles et au moins un groupement hydroxyle et/ou amine,

(ii) les acides phosphoriques organiques de formules suivantes :

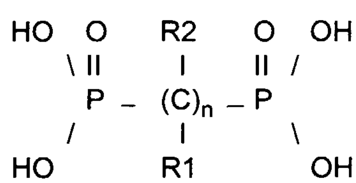

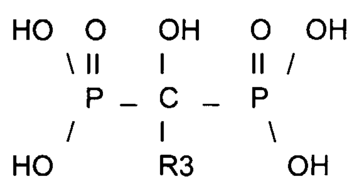

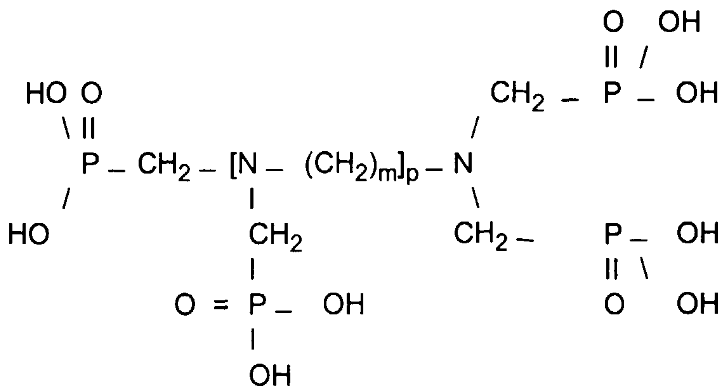

dans lesquelles, n et m sont des nombres entiers compris entre 1 et 6, p est un nombre entier compris entre 0 et 5, R1, R2, R3 identiques ou différents représentant un groupement hydroxyle, amino, aralkyl, aryl, alkyl ou l'hydrogène,

(iii) les composés capables de libérer des ions sulfates en milieu acide,

(iv) les sels des acides décrits ci-dessus,

et en présence de germes de dioxyde de titane anatase présentant une taille d'au plus 5 nm et dans un rapport pondéral exprimé en $TiO_2$ présent dans les germes/titane présent avant introduction des germes dans le milieu d'hydrolyse, exprimé en $TiO_2$ compris entre 0,01 % et 3 %.

**13.** Procédé selon la revendication 12, caractérisé en ce que le composé du titane A est l'oxychlorure de titane.

**14.** Procédé selon la revendication 12 ou 13, caractérisé en ce que le composé B est l'acide citrique.

**15.** Utilisation des particules selon l'une des revendications 1 à 9 en tant qu'agent anti-UV.

**16.** Utilisation selon la revendication 15 dans les formulations pour cosmétiques, vernis, peintures et dans les plastiques.

**17.** Composition cosmétique anti-UV, caractérisée en ce qu'elle comprend des particules de dioxyde de titane selon l'une des revendications 1 à 10, en quantité telle que la teneur de ladite composition en dioxyde de titane est d'au moins 1 %, de préférence d'au plus 25 % en poids.

**Claims**

**1.** Anatase titanium dioxide particles with a size of at most 100 nm, characterized in that said particles are at least partially coated with a layer of at least one metal oxide, hydroxide or oxohydroxide and in that these particles have a BET specific surface area of at least 70 $m^2/g$ and a density of the order of 2.2.

**2.** Particles according to claim 1, characterized in that the ratio of the metal oxide(s), hydroxide(s) or oxohydroxide (s) to the titanium dioxide is at most 60% by weight.

**3.** Particles according to claim 1 or claim 2, characterized in that they are at least partially coated with a layer of silica or an aluminium hydroxide or oxohydroxide in the simple or mixed form.

**4.** Particles according to claim 2, characterized in that they are coated with a layer of silica and aluminium hydroxide or oxohydroxide in amounts of 15% of $SiO_2$ and 5% of $Al_2O_3$ with respect to the titanium dioxide.

**5.** Particles according to any one of the preceding claims, characterized in that they are in the form of a dispersion.

**6.** Particles according to claim 5, characterized in that the dispersion index of the particles in the liquid phase is at most 0.5.

**7.** Particles according to claim 5 or claim 6, characterized in that the liquid phase of the dispersion is aqueous.

**8.** Particles according to claim 7, characterized in that the conductivity of the dispersion is at most 3 msiemens.

**9.** Particles according to any one of claims 5 to 7, characterized in that the dry extract of the dispersion is at least 35% by weight and in that the viscosity of said dispersion is at most 1000 mPa.s.

**10.** Particles according to any one of claims 1 to 4, characterized in that they are agglomerated and are in the form of a powder.

**11.** A process for preparing particles according to any one of claims 1 to 10, characterized in that at least one metal oxide, hydroxide or oxohydroxide is precipitated onto the surface of anatase titanium dioxide with a size of at most 100 nm, with a BET specific surface area of at least 200 $m^2/g$ and with a density of the order of 2.5.

**12.** A preparation process according to claim 11, characterized in that at least one metal oxide, hydroxide or oxohydroxide is precipitated onto the surface of titanium dioxide particles obtained by hydrolysis of at least one titanium compound A in the presence of at least one compound B selected from:

(i) acids having:

- either a carboxyl group and at least two hydroxyl and/or amine groups;
- or at least two carboxyl groups and at least one hydroxyl and/or amine group;

(ii) organic phosphoric acids with the following formulae:

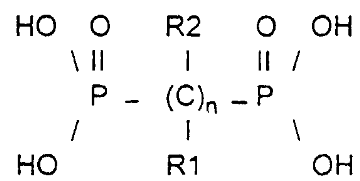

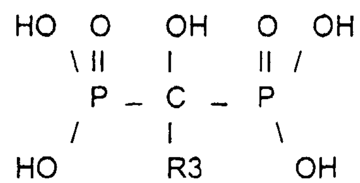

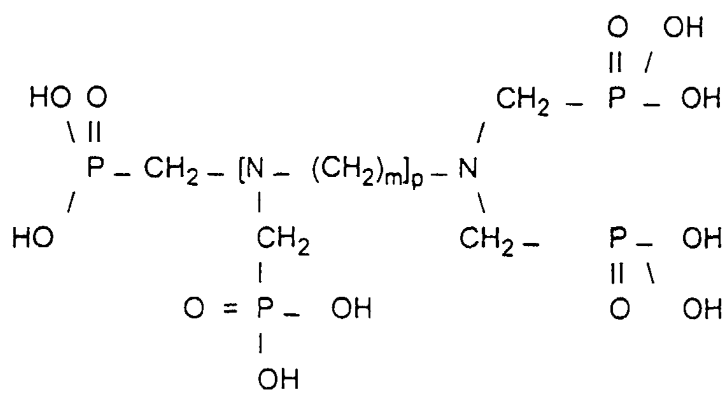

where n and m are whole numbers in the range 1 to 6, p is a whole number in the range 0 to 5, and R1, R2 and R3, which may be identical or different, represent a hydroxyl, amine, aralkyl, aryl or alkyl group, or hydrogen;
(iii) compounds which are capable of liberating sulphate ions in an acid medium;
(iv) salts of the acids defined above;

and in the presence of seeds of anatase titanium dioxide with a size of at most 5 nm and in a weight ratio, expressed as the $TiO_2$ present in the seeds/titanium present before introducing the seeds into the hydrolysis medium, expressed as $TiO_2$, in the range 0.01% to 3%.

**13.** A process according to claim 12, characterized in that titanium compound A is titanium oxychloride.

**14.** A process according to claim 12 or claim 13, characterized in that compound B is citric acid.

**15.** Use of particles according to any one of claims 1 to 9 as an anti-UV agent.

**16.** Use according to claim 15 in formulations for cosmetics, lacquers, paints and plastics.

**17.** A cosmetic anti-UV composition, characterized in that it comes particles of titanium dioxide in accordance with any one of claims 1 to 10, in a quantity such that the amount of said titanium dioxide composition is at least 1%, preferably at most 25% by weight.

**Patentansprüche**

**1.** Teilchen von Titandioxid Anatas mit einer Größe von höchstens 100 nm, dadurch gekennzeichnet, daß diese Teilchen mindestens teilweise von einer Schicht von mindestens einem metallischen Oxid, Hydroxid oder Oxohydroxid bedeckt sind, und dadurch, daß diese Teilchen eine spezifische Oberfläche BET von mindestens 70 $m^2/g$ und eine Dichte in der Größenordnung von 2,2 aufweisen.

**2.** Teilchen nach Anspruch 1, dadurch gekennzeichnet, daß das Gewichtsverhältnis des oder der metallischen Oxide, Hydroxide oder Oxohydroxide zum Titandioxid höchstens 60 Gew.-% beträgt.

**3.** Teilchen nach Anspruch 1 oder 2, dadurch gekennzeichnet, daß sie mindestens teilweise von einer Schicht von Siliciumdioxid oder einem Hydroxid oder Oxohydroxid von Aluminium in einfacher oder gemischter Form bedeckt sind.

**4.** Teilchen nach Anspruch 2, dadurch gekennzeichnet, daß sie mit einer Schicht von Siliciumdioxid und Hydroxid oder Oxohydroxid von Aluminium in Gewichtsverhältnissen von 15 % $SiO_2$ und 5 % $Al_2O_3$ bedeckt sind, bezogen auf das Titandioxid.

**5.** Teilchen nach irgendeinem der vorstehenden Ansprüche, dadurch gekennzeichnet, daß sie in Form einer Dispersion vorliegen.

**6.** Teilchen nach Anspruch 5, dadurch gekennzeichnet, daß ihr Dispersionsindex der Teilchen in der flüssigen Phase höchstens 0.5 beträgt.

**7.** Teilchen nach Anspruch 5 oder 6, dadurch gekennzeichnet, daß die flüssige Phase der Dispersion wäßrig ist.

**8.** Teilchen nach Anspruch 7, dadurch gekennzeichnet, daß die Dispersion eine Leitfähigkeit von höchstens 3 mSiemens aufweist.

**9.** Teilchen nach irgendeinem der Ansprüche 5 bis 7, dadurch gekennzeichnet, daß der Trockenextrakt der Dispersion mindestens 35 Gew.-% und daß die Viskosität der genannten Dispersion höchstens 1000 mPa.s betragen.

**10.** Teilchen nach einem der Ansprüche 1 bis 4, dadurch gekennzeichnet, daß sie agglomeriert sind und in Form eines Pulvers vorliegen.

**11.** Verfahren zur Herstellung der Teilchen nach einem der Ansprüche 1 bis 10, dadurch gekennzeichnet, daß man mindestens ein metallisches Oxid, Hydroxid oder Oxohydroxid auf der Oberfläche der Teilchen von Titandioxid

Anatas mit einer Größe von höchstens 100 nm ausfällt, die eine spezifische Oberfläche BET von mindestens 200 $m^2$/g und eine Dichte in der Größenordnung von 2,5 aufweisen.

**12.** Verfahren zur Herstellung nach Anspruch 11, dadurch gekennzeichnet, daß man mindestens ein metallisches Oxid, Hydroxid oder Oxohydroxid auf der Oberfläche der Teilchen von Titandioxid ausfällt, erhalten durch Hydrolyse von mindestens einer Verbindung von Titan A in Anwesenheit von mindestens einer Verbindung B, ausgewählt unter:

(i) den Säuren, die aufweisen:

- entweder eine Gruppe Carboxyl und mindestens zwei Gruppen Hydroxyl und/oder Amin,
- oder mindestens zwei Gruppen Carboxyl und mindestens eine Gruppe Hydroxyl und/oder Amin,

(ii) den organischen Phosphorsäuren der folgenden Formeln:

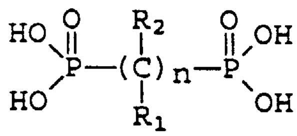

;

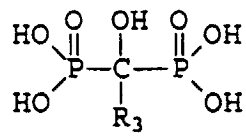

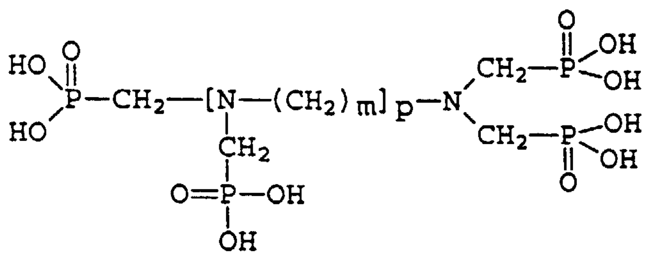

worin n und m ganze Zahlen zwischen 1 und 6 sind, p eine ganze Zahl zwischen 0 und 5 ist, $R_1$, $R_2$, $R_3$, gleich oder verschieden, eine Gruppe Hydroxyl, Amino, Aralkyl, Aryl, Alkyl oder Wasserstoff darstellen,
(iii) den Verbindungen, die fähig sind, im sauren Medium Sulfationen freizusetzen,
(iv) den Salzen der oben genannten Säuren,

und in Anwesenheit von Keimen von Titandioxid Anatas mit einer Größe von höchstens 5 nm und in einem Gewichtsverhältnis, ausgedrückt in $TiO_2$, das in den Keimen vorliegt/Titan, das vor dem Eintragen der Keime in dem Hydrolysemedium vorliegt, ausgedrückt in $TiO_2$, zwischen 0,01 % und 3 %.

**13.** Verfahren nach Anspruch 12, dadurch gekennzeichnet, daß die Verbindung von Titan A Titanoxychlorid ist.

**14.** Verfahren nach Anspruch 12 oder 13, dadurch gekennzeichnet, daß die Verbindung B Citronensäure ist.

**15.** Verwendung der Teilchen nach einem der Ansprüche 1 bis 9 als Anti-UV-Mittel.

**16.** Verwendung nach Anspruch 15 in Formulierungen für Kosmetika, Lacke, Anstrichstoffe und in Kunststoffen.

**17.** Kosmetische Anti-UV-Zusammensetzung, dadurch gekennzeichnet, daß sie Teilchen von Titandioxid nach einem der Ansprüche 1 bis 10 in solcher Menge umfaßt, daß der Gehalt der genannten Zusammensetzung an Titandioxid mindestens 1 %, vorzugsweise höchstens 25 Gew.-% beträgt.